# EUROPEAN PATENT APPLICATION

(11) **EP 3 695 856 A1**
(43) Date of publication of application: **19.08.2020**
(21) Application number: 19305180.2
(22) Date of filing: 13.02.2019
(51) Int. Cl.: A61L 27/12, A61L 27/34, A61L 27/46, A61L 27/54, A61L 27/38

(54) **BIOMATERIALS COMPRISING A SCAFFOLD CONTAINING A MINERAL COMPOUND, AND USES THEREOF AS BONE SUBSTITUTES**

(71) Applicant: INSTITUT NATIONAL DE LA SANTE ET DE LA RECHERCHE MEDICALE (INSERM), 75013 Paris (FR); Université de Strasbourg, 67000 Strasbourg (FR)
(72) Inventor: BORNERT, Fabien, 67210 OBERNAI (FR); HUCK, Olivier, 67100 STRASBOURG (FR); OFFNER, Damien, 67000 STRASBOURG (FR); IDOUX-GILLET, Ysia, 67100 STRASBOURG (FR); CLAUSS, François, 67380 LINGOLSHEIM (FR); BENKIRANE-JESSEL, Nadia, 67270 KIENHEIM (FR)
(74) Representative: Lavoix

(57) **Abstract**

The present invention concerns a biomaterial comprising a scaffold containing a mineral component, wherein said mineral component comprises at least one calcium phosphate compound,
and wherein said scaffold has a surface coated with an interrupted coating made of multilayered droplets, said multilayered droplets being droplets composed of at least one layer pair consisting of a layer of polyanions and a layer of polycations.

## Description

The present invention concerns biomaterials comprising a scaffold containing a mineral compound and nanoreservoirs on its surface, as well as uses thereof, in particular as bone substitutes.

Bone substitute materials is a domain of the bone tissue engineering aiming to replace and overcome the critical limitations associated to autologous bone graft, currently accepted as the standard therapy in orthopaedic surgery and traumatology (Campana V, et al. Bone substitutes in orthopaedic surgery: from basic science to clinical practice. J Mater Sci Mater Med 25, 2445-2461 (2014); Kumar Saper et al, Korean J Intern Med. 2015 May; 30(3): 279-293; Pryor LS, et al. Review of bone substitutes. Craniomaxillofac Trauma Reconstr 2, 151-160 (2009); Roberts TT, Rosenbaum AJ. Bone grafts, bone substitutes and orthobiologics: the bridge between basic science and clinical advancements in fracture healing. Organogenesis 8, 114-124 (2012)). These last years, innovative bone substitutes were developed based on biphasic biomimetism principle, and occupy an important place in the orthopaedic surgery market. Mimicking the natural composition of the bone tissue, these biomaterials contain an organic phase (composed with collagen or with polymers) in order to offer the best conditions of proliferation, infiltration and differentiation for cells (V. Campana, G. Milano, E. Pagano, M. Barba, C. Cicione, G. Salonna, W. Lattanzi, and G. Logroscino, 2014 J Mater Sci Mater Med. 2014; 25(10): 2445-2461; Kumar Saper et al, Korean J Intern Med. 2015 May; 30(3): 279-293; Pryor et al., Trauma Reconstr. 2009 Oct; 2(3): 151-160; Roberts et al., Organogenesis. 2012 Oct 1; 8(4): 114-124). This organic part is associated to a mineral phase (ceramics, hydroxyapatite (Hap), tricalcium phosphate (TCP)) allowing to the bone defect filling and substituting to the function of bone tissue in term of resistance against constraints applied by mechanical strengths. Success of tissue engineering construct is limited by vascularization as it provides essentials nutrients and oxygenation of the tissue (Kaully T, Kaufman-Francis K, Lesman A, Levenberg S. Vascularization--the conduit to viable engineered tissues. Tissue Eng Part B Rev 15, 159-169 (2009); Baiguera S, Ribatti D. Endothelialization approaches for viable engineered tissues. Angiogenesis 16, 1-14 (2013); Brennan MA, et al. Pre-clinical studies of bone regeneration with human bone marrow stromal cells and biphasic calcium phosphate. Stem Cell Res Ther 5, 114 (2014); Mertsching H, Walles T, Hofmann M, Schanz J, Knapp WH. Engineering of a vascularized scaffold for artificial tissue and organ generation. Biomaterials 26, 6610-6617 (2005); Khan OF, Sefton MV. Endothelialized biomaterials for tissue engineering applications in vivo. Trends Biotechnol 29, 379-387 (2011); Mao AS, Mooney DJ. Regenerative medicine: Current therapies and future directions. Proc Natl Acad Sci U S A 112, 14452-14459 (2015)). Currently, failures of bone substitutes applications, caused by non-integration to host tissues and necrosis are linked to lack of vascularization, especially in case of large bone injuries (Mercado-Pagan AE, Stahl AM, Shanjani Y, Yang Y. Vascularization in bone tissue engineering constructs. Ann Biomed Eng 43, 718-729 (2015); Brennan et al., 2013 stem cell research*;* Baiguera et al. 2013; Boerckel JD, Uhrig BA, Willett NJ, Huebsch N, Guldberg RE. Mechanical regulation of vascular growth and tissue regeneration in vivo. Proc Natl Acad Sci U S A 108, E674-680 (2011); Tsigkou et al., Proc. Natl. Acad. Sci. U.S.A. 107, 3311-3316 (2010)). This could be explained by the fact that invasion of implants by host vasculature occur with approximately 10 µm per days and several weeks could then be necessary to vascularize an implant of 3-4 mm (Baiguera et al., 2013). Nowadays, treatments of large bone defects still remain challenge for current medical practitioner.

The aim of the present invention is to provide a new biomaterial suitable as bone substitute with suitable vascularization properties.

The aim of the present invention is also to provide a new hybrid bone substitute, comprising both a mineral component and a polymeric component, said bone substitute being efficient for the sustained release of angiogenic factors.

Another aim of the present invention is to provide a new biomaterial including nanoreservoirs allowing a cell contact-dependent release of growth factors, preventing passive release after implantation and avoiding side effects caused by a too local dose delivery of active molecules.

Another aim of the present invention is also to provide a new biomaterial suitable for the treatment of large bone defects.

Thus, the present invention relates to a biomaterial comprising a scaffold containing a mineral component,
wherein said mineral component comprises at least one calcium phosphate compound, and
wherein said scaffold has a surface coated with an interrupted coating made of multilayered droplets, said multilayered droplets being droplets composed of at least one layer pair consisting of a layer of polyanions and a layer of polycations.

According to an embodiment, the scaffold of the biomaterial of the invention further contains a polymeric component.

By "biomaterial" is meant any material suitable for use *in vivo* in mammals, in particular in human patients. More specifically, the biomaterials according to the invention are suitable for use as implants.

As mentioned above, the scaffold of the biomaterial according to the invention comprises a mineral component, and optionally also a polymeric component.

### Mineral component

The mineral component comprises at least one calcium phosphate compound, said calcium phosphate compound being preferably selected from the group consisting of: hydroxyapatite (HA), amorphous calcium phosphate (ACP), monocalcium phosphate anhydrous (MCPA), monocalcium phosphate monohydrate (MCPM), dicalcium phosphate dihydrate (DCPD), dicalcium phosphate anhydrous (DCPA), precipitated or calcium-deficient apatite (CDA), β-tricalcium phosphate (β-TCP), tetracalcium phosphate (TTCP), and mixtures thereof.

According to an embodiment, the mineral component comprises hydroxyapatite.

According to an embodiment, the mineral component comprises β-tricalcium phosphate.

According to a preferred embodiment, the mineral component comprises a mixture of hydroxyapatite and β-tricalcium phosphate.

### Polymeric component

According to an embodiment, the scaffold also comprises a polymeric component. According to the invention, the polymeric component is made of a polymer.

Preferably, the polymeric component is made of a polymer chosen from the group consisting of: poly(ε-caprolactone), collagen, fibrin, poly(lactic acid), poly(glycolic acid), poly(ethylene glycol)-terephtalate, poly(butylenes terephtalate), or co-polymers thereof, and mixtures thereof.

When the scaffold contains a mineral component together with a polymeric component, the multilayered droplets as defined above which coat the surface of the scaffold are present at the surface of the mineral component but also at the surface of the polymeric part.

The presence of the multilayered droplets on both parts of the scaffold is an advantageous feature of the biomaterial of the invention and thus gives a hybrid biomaterial with advantageous properties.

### Multilayered droplets

As mentioned above, the scaffold of the invention has a surface coated with an interrupted coating of multilayered droplets. These droplets may also be named "nanoreservoirs" or "nanocontainers".

The inventors have surprisingly found that it is possible to coat the scaffold (both its mineral component and its polymeric component) with at least one layer pair consisting of:
- a layer of polyanions; and
- a layer of polycations.

According to some embodiments, the biomaterial scaffold is multilayered droplet coated.

The coating according to the invention is preferably, irregularly spread over the scaffold surface.

More specifically, the biomaterial scaffold according to the invention is coated, on a layer-by-layer basis, with layers that are alternatively negatively or positively charged.

This coating allows functionalizing the biomaterial scaffold with a therapeutic molecule in such a way as to create nano-reservoirs of therapeutic molecules, as explained hereafter.

The term "multilayered droplet" refers to droplets or patches composed of at least one layer pair consisting of a layer of polyanions and a layer of polycations. Said droplets can present different shapes: circle shaped, oval-shaped or scale shaped. Preferably said droplets have a size of 10 to 150 nm, more preferably 15 to 100 nm, even more preferably 25 to 50 nm.

According to the invention, the term "multilayered droplet coating" refers to a coating of droplets or patches disposed at the surface of the scaffold and obtained by layer-by-layer (LbL) deposition of oppositely charged molecules multilayered droplet.

The term "multilayered droplet coating" further refers to an interrupted coating of the scaffold, i.e. a coating that is not in the form of a continuous film along the surface of the biomaterial scaffold. The multilayer droplet coating may be characterized by its irregular shape and/or by the fact that it does not cover the totality of the surface of the scaffold, in such a way that at least a part of the surface of the scaffold is not coated. The multilayer droplet coating of the invention may be contrasted with a film coating having a smooth surface and covering the totality of the scaffold surface.

The building of the coating is based on the layer-by-layer (LbL) deposition of oppositely charged molecules. That is to say, the coating of the biomaterial scaffold is made in the same manner as is made a polyelectrolyte multilayered film. The biomaterial according to the invention thus comprises polyelectrolyte multilayers, in the form of numerous multilayered droplets, on the surface of the biomaterial scaffold.

In contrast to a film coating that covers the whole scaffold surface, the multilayered droplet coating according to the invention preferably only partially covers the scaffold surface. The coating according to the invention is applied layer by layer (LbL), the excess amount of polyanions or polycations is removed at each step with rinsing steps between consecutive adsorption steps. Due to the repartition of the surface charges, the first layer of polyanions or polycations form small droplets or patches adsorbed along the surface of the scaffold. At each step of the polyanions or polycations application, each droplet is covered by a new layer of polyanions or polycations. The coating process is stopped when the multilayered droplet coating is observed and before a film coating. The multilayered droplet coating provides advantageous characteristics to the scaffold, which are not observed with a film coating. When the film coating is obtained, the multilayered droplets cannot be obtained any more along the surface of the coated scaffold.

The first advantage of the multilayered droplet coating compared with the film coating or the uncoated scaffold is its irregular surface. This irregular shape improves the adherence of cells to the scaffold. Moreover, this irregular shape provides an increase of the surface of contact between the coating and cells, optimizing the exchanges between the coating and cells. Consequently, a small concentration of therapeutic molecule (if present) is needed for observing a better stimulation of cell growth.

In addition, the coating of the invention uses fewer polyanions and polycations layers than the film coating. A reduced number of layers are thus needed to obtain the multilayered droplet coating.

As further used herein, the term "polyelectrolyte multilayers" notably encompasses the multilayered droplets that coat the biomaterial scaffold according to the invention.

In the frame of the present specification, the term "polyelectrolyte" designates compounds that bear several electrolyte groups, in particular polymers whose repeating units carry electrolyte groups. The groups will dissociate in aqueous solutions, giving rise to polyanions or polycations, as the case may be, and making the polymers charged.

The polyelectrolyte multilayers that coat the nanofibrous scaffold are composed of at least one layer pair consisting of a layer of polyanions and of a layer of polycations. They may for example comprise 1, 2, 3, 4, 5, 6, 7, 8, 9, 10, 11, 12, 13, 14, 15 or more layer pairs. Preferably, it comprises from 3 to 12 layer pairs.

Polyelectrolyte multilayers, and in particular multilayered droplet as described herein, can easily be obtained by the alternate dipping of the biomaterial scaffold in polyanion and polycation solutions.

As apparent to the skilled in the art, the only requirement for the choice of the polyanions and polycations is the charge of the molecule, i.e., the polyanion shall be negatively charged and the polycation shall be positively charged. The polyanions and polycations according to the invention may correspond to any type of molecule, such as e.g. a polypeptide (optionally chemically modified) or a polysaccharide (including cyclodextrins, chitosan, *etc*.).

According to a preferred embodiment, in the biomaterial of the invention, the polycations are chosen from the group consisting of: poly(lysine) polypeptides (PLL), covalently-coupled cyclodextrin-poly(lysine) (PLL-CDs), poly(arginine) polypeptides, poly(histidine) polypeptides, poly(ornithine) polypeptides, Dendri-Graft Poly-lysines (e.g. Dendri-Graft Poly-L-lysines), chitosan, and mixtures thereof.

More preferably, the polycation is chitosan.

According to a preferred embodiment, in the biomaterial of the invention, the polyanions are chosen from the group consisting of: poly(glutamic acid) polypeptides (PGA), poly(aspartic acid) polypeptides, and mixtures thereof.

### Therapeutic molecule

According to an embodiment, the biomaterial of the invention further comprises a therapeutic molecule within at least one multilayered droplet or forming at least one multilayered droplet when said therapeutic molecule is charged.

According to an embodiment, as explained above, at least one layer pair of the multilayered droplets incorporates the therapeutic molecule.

The polyelectrolyte multilayers that coat the biomaterial scaffold may incorporate a therapeutic molecule or one of the polyelectrolyte multilayers may be the therapeutic molecule.

When the therapeutic molecule to be incorporated to the biomaterial according to the invention is charged, said therapeutic molecule may be used as a polyanion or as a polycation when building the polyelectrolyte multilayers. When the therapeutic molecule is not charged, or not sufficiently charged, it may be covalently linked with a polyanion or a polycation (e.g. one of those listed above) in order to build the polyelectrolyte multilayers.

According to a preferred embodiment, in the biomaterial of the invention, the polyanion is the therapeutic molecule, which is in particular VEGF.

Preferably, the therapeutic molecule is a growth factor selected from the group consisting of: a vascular endothelial growth factor (VEGF), a bone morphogenetic protein (BMP), a transforming growth factor (TGF), a fibroblast growth factor (FGF), a nucleic acid coding therefor, and mixtures thereof.

Therapeutic molecules can be incorporated into polyelectrolyte multilayers, as described, e.g., in WO 02/085423, WO 2006/079928, Lynn (2006 Soft Matter 2:269-273), Decher (1997 Science 277:1232-1237) and Jessel et al. (2003 Advanced Materials 15:692-695).

In the frame of the present invention, the biomaterial scaffold may be functionalized with a therapeutic molecule, allowing sustained release of said therapeutic molecule at the site of implantation of the biomaterial according to the invention.

As used throughout the present specification, the term "therapeutic molecule" refers to any molecule intended to treat or prevent a disease. It may for example correspond to a drug for which a marketing approval has been issued (e.g. by the European Medicines Agency (EMA) or by the U.S. Food and Drug Administration (FDA)), or to a candidate drug undergoing clinical or pre-clinical trials. The therapeutic molecule may for example correspond to a polypeptide (including recombinant proteins, antibodies and peptides), a nucleic acid (including RNA and DNA molecules), a chemical molecule (e.g. a small molecule), or a sugar (e.g. a lipopolysaccharide).

When the biomaterial according to the invention is used for bone and/or cartilage regeneration, said growth factor is most preferably selected from the group consisting of bone morphogenetic protein 2 (BMP2), bone morphogenetic protein 4 (BMP4), bone morphogenetic protein 7 (BMP7), fibroblast growth factor 1 (FGF1), fibroblast growth factor 2 (FGF2), fibroblast growth factor 4 (FGF4), fibroblast growth factor 8 (FGF8), fibroblast growth factor 9 (FGF9) and fibroblast growth factor 18 (FGF18).

In a specific embodiment, the polyelectrolyte multilayers comprise or consist of at least 1, 2, 3, 4, 5, 6, 7, 8, 9, 10, 11, 12, 13, 14, 15 or more layer pairs, each layer pair consisting of:
- a layer of polyanions comprising or consisting of the therapeutic molecule (such as e.g. a polypeptide, in particular a growth factor); and
- a layer of polycations comprising or consisting of chitosan or of a polymer of lysines (such as e.g. a poly(lysine) polypeptide (PLL) or a Dendri-Graft poly-lysine (DGLs)).

### Living cells

According to an embodiment, the biomaterial of the invention further comprises living cells.

In the context of the present invention, the biomaterial scaffold may further be functionalized with living cells. Indeed, implanting living cells is a promising solution to tissue or organ repair.

In the context of bone and/or cartilage regeneration, said living cells may for example comprise or consist of osteoblasts, chondrocytes, stem cells (e.g. mesenchymal stem cells), bone marrow stromal cells, or a mixture thereof. Preferably, said living cells comprise or consist of osteoblasts, chondrocytes, or a mixture thereof. In a specific embodiment, embryonic stem cells may be excluded from the living cells according to the invention.

Said living cells are preferably human cells, and most preferably autologous cells (i.e. cells that are obtained from the patient to be treated).

Said living cells are preferably obtained by induced pluripotent stem cells (iPSCs) technology.

In a specific embodiment, said living cells are comprised within a hydrogel (e.g. an alginate hydrogel or a collagen hydrogel) that is deposited on said coated scaffold. In other terms, the biomaterial according to the invention may comprise, in addition to the coated scaffold, a hydrogel comprising living cells.

Hydrogels are well-known to the skilled in the art. A collagen hydrogel may for example be prepared by mixing collagen (e.g. 3 mL of Rat Tail Type-I Collagen) with a medium containing 10% FBS (e.g. 5.5. mL) and with a 0.1 M NaOH solution (e.g. 0.5. mL). An alginate hydrogel may for example be a mixture of alginate and hyaluronic acid (e.g. a alginate:hyaluronic acid solution (4:1), which may be prepared in a 0.15 M NaCl solution at pH 7.4).

In a preferred embodiment according to the invention, the biomaterial according to the invention comprises or consists of:
- the scaffold that is coated with at least one layer pair consisting of a layer of polyanions and a layer of polycations; and
- osteoblasts that are optionally comprised within a collagen hydrogel (deposited on said coated scaffold).

In another preferred embodiment, the biomaterial according to the invention comprises or consists of:
- the scaffold that is coated with at least one layer pair consisting of a layer of polyanions and a layer of polycations;
- osteoblasts that are optionally comprised within a collagen hydrogel (deposited on said coated scaffold); and
- chondrocytes comprised within an alginate hydrogel (deposited on said coated scaffold).

In still another preferred embodiment according to the invention, the biomaterial according to the invention comprises or consists of:
- the scaffold that is coated with at least one layer pair consisting of a layer of polyanions and a layer of polycations;
- chondrocytes comprised within an alginate hydrogel (deposited on said coated nanofibrous scaffold).

In still another preferred embodiment according to the invention, the biomaterial according to the invention does not comprise living cells. More specifically, it may simply consist of the scaffold as defined above that is coated with at least one layer pair consisting of a layer of polyanions and a layer of polycations.

### Method for preparing the biomaterial

The present invention also relates to a method for preparing the biomaterial as defined above, said method comprising a step of coating a scaffold containing a mineral component and an optional polymeric component with at least one layer pair consisting of a layer of polyanions and a layer of polycations.

Preferably, the above-mentioned step of coating with at least one layer pair comprises the following steps:
i. immersing the scaffold in a solution comprising the polycations (e.g. during about 5 to 60 min, preferably during about 15 min);
ii. rinsing the scaffold obtained at the end of step (i) (e.g. during about 5 to 60 min, preferably during about 15 min);
iii. immersing the scaffold obtained at the end of step (ii) in a solution comprising the polyanions (e.g. during about 5 to 60 min, preferably during about 15 min);
iv. rinsing the scaffold obtained at the end of step (iii) (e.g. during about 5 to 60 min, preferably during about 15 min); and, optionally;
v. repeating step (i) to (iv) for at least a second time; and, optionally;
vi. sterilizing the scaffold obtained at the end of step (iv) or (v) (e.g. by exposure to ultraviolet light).

At step (i) and (iii), the solution comprising the polycations or polyanions may for example comprise a concentration of polycations or polyanions within a range of about 20 µM to about 500 µM, preferably of about 50 µM to about 200 µM. Said solution may for example comprise or consist of, in addition to the polyanions or polycations, 0.02 M 2-(N-morpholino)ethanesulfonic acid (MES) and 0.15M NaCl. The pH of the solution is preferably neutral (e.g. a pH of 7.4).

At step (ii) and (iv), the scaffolds may for example be rinsed with a solution having a neutral pH (e.g. a pH of 7.4). Said solution may for example comprise or consist of 0.02 M MES and 0.15 M NaCl.

Step (v) may be repeated any number of times, depending on the number of layer pairs that should coat the scaffold.

Step (vi) may for example be carried out by exposure to ultraviolet light (for example at 254 nm, 30 W, at an illumination distance of 20 cm, for about 15 min to about 1 hour, preferably for about 30 min).

Before use, the biomaterial according to the invention may be equilibrated (e.g. by bringing it in contact with serum-free medium).

As immediately apparent to the skilled in the art, the steps in which the nanofibrous scaffold is immersed in a solution comprising polycations or polyanions may be replaced with steps wherein said solution is sprayed onto the scaffold.

The above method for producing the biomaterial according to the invention may further comprise the steps of:
a) providing or obtaining living cells (e.g. osteoblatsts or chondrocytes isolated from a patient suffering from a bone and/or cartilage defect);
b) mixing said living cells with a hydrogel (e.g. a collagen hydrogel or an alginate hydrogel); and
c) depositing the mixture obtained at step (d) on the biomaterial obtained at step (b).

Methods for preparing hydrogels are well-known to the skilled in the art. The collagen hydrogel may for example be prepared by mixing collagen (e.g. 3 mL of Rat Tail Type-I Collagen) with a medium containing 10% FBS (e.g. 5.5. mL) and with a 0.1 M NaOH solution (e.g. 0.5. mL). The alginate hydrogel may for example be a mixture of alginate and hyaluronic acid (e.g. an alginate:hyaluronic acid solution (4:1), which may be prepared in a 0.15 M NaCl solution at pH 7.4).

In a specific embodiment, the living cells are osteoblasts, and the hydrogel is a collagen hydrogel. In the frame of this embodiment, step (d) may be carried out by mixing an osteoblast suspension (e.g. at 2x10⁵ cells.mL⁻¹) with the collagen hydrogel (e.g. 1 mL osteoblast suspension mixed with 9 mL of hydrogel). At step (e), the collagen preparation can be poured on the top of the biomaterial obtained at step (b), and may then be incubated in order to allow polymerization (e.g. at 37°C for about 30 min).

In another specific embodiment, the living cells are chondrocytes, and the hydrogel is an alginate hydrogel. In the frame of this embodiment, step (d) can be performed by mixing a chondrocyte suspension (e.g. at 1x10⁵ cells.mL⁻¹) with the alginate hydrogel. At step (e), this preparation can be poured on the top of the biomaterial obtained at step (b).

Before use, cylinders can be cut (e.g. using a sterile biopsy punch), and incubated at about 37°C, e.g. overnight in a humidified atmosphere of 5% CO₂.

Alternatively, the living cells may also be directly deposited on the coated scaffold obtained at step (b) or (e), without previous mixture with a hydrogel.

When both osteoblasts and chondrocytes should be deposited on the coated scaffold according to the invention, the above method for producing the biomaterial according to the invention may further comprise, after steps (a) and (b), the steps of:
c) providing or obtaining osteoblatsts (e.g. isolated from a patient suffering from a bone and/or cartilage defect);
d) optionally mixing said osteoblatsts with a collagen hydrogel;
e) depositing the osteoblasts obtained at step (c) or the mixture obtained at step (d) on the biomaterial obtained at step (b);
f) providing or obtaining chondrocytes (e.g. isolated from a patient suffering from a bone and/or cartilage defect);
g) mixing said chondrocytes with a alginate hydrogel; and
h) depositing the mixture obtained at step (g) on the biomaterial obtained at step (e).

These steps (c) to (h) can for example be carried out as described in detail hereabove.

The invention further provides biomaterials obtainable by the methods described herein.

### Uses

The present invention also relates to the use of the biomaterial as defined above as a bone substitute.

The present invention also relates to the biomaterial as defined above, for use as a bone and/or cartilage defect filling material, or for use in bone and/or cartilage regeneration.

The present invention also relates to the biomaterial as defined above, for use in the treatment of a bone and/or cartilage defect.

The bone and/or cartilage defect may affect either the bone, or the cartilage, or both. It may for example be a chondral defect, an osteochondral defect, or a subchondral bone defect.

In a specific embodiment according to the invention, the bone and/or cartilage defect is a subchondral bone defect. The invention thus provides a biomaterial described in the above paragraphs for use in subchondral bone regeneration and/or for use in the treatment of a subchondral bone defect.

The invention also provides a biomaterial described in the above paragraphs for use in osteochondral bone regeneration and/or for use in the treatment of a osteochondral bone defect.

In particular, the biomaterial according to the invention finds use in the treatment of bone and/or cartilage defect(s) in patients suffering from osteochondritis dissecans, osteonecrosis, osteochondral fracture(s), spinal fusion, a bone and/or cartilage defect due to an injury (e.g. a sport injury or an injury due to an accident), a bone and/or cartilage defect due to ageing, a bone and/or cartilage defect necessitating maxillofacial reconstruction, a bone and/or cartilage defect necessitating sinus lift, a bone and/or cartilage defect necessitating alveolar ridge augmentation, or bone and/or cartilage loss due to a tumor (including benign and cancerous tumors).

In a specific embodiment, the bone and/or cartilage defect is an articular defect, such as e.g. a defect of the knee and/or of the ankle.

In the frame of bone and/or cartilage repair and regeneration, the biomaterial according to the invention may or may not comprise living cells. When it comprises living cells, the cells are preferably autologous cells, i.e. cells isolated from the patient to be treated. As indicated hereabove, these living cells may be comprised within a hydrogel.

When the biomaterial is for use as an implant in the treatment of a small bone and/or cartilage defect (e.g. in the frame of maxillofacial or orthopedic surgery), the biomaterial may be devoid of living cells.

On the other hand, when the bone and/or cartilage defect is a large and/or deep defect, it is preferred that the biomaterial comprises living cells. For instance, when the biomaterial is for use as an implant in the treatment of a large and/or deep bone defect, the biomaterial preferably comprises osteoblasts. When the biomaterial is for use as an implant in the treatment of a large and/or deep cartilage defect, the biomaterial preferably comprises chondrocytes. When the biomaterial is for use as an implant in the treatment of large and/or deep defects affecting the bone and the cartilage (e.g. an osteochondral defect or a subchondral bone defect), the biomaterial preferably comprises both osteoblasts and chondrocytes.

In a preferred embodiment according to the invention, the biomaterial according to the invention comprises or consists of:
- the scaffold that is coated with at least one layer pair consisting of a layer of polyanions and a layer of polycations; and
- osteoblasts that are optionally comprised within a collagen hydrogel (deposited on said coated scaffold);
and is for use in bone regeneration, and/or in the treatment of a bone defect (preferably a deep and/or large bone defect). Indeed, such a biomaterial is particularly well-suited for the treatment of defects only affecting the bone but not the cartilage.

In another preferred embodiment according to the invention, the biomaterial according to the invention comprises or consists of:
- the scaffold that is coated with at least one layer pair consisting of a layer of polyanions and a layer of polycations;
- osteoblasts that are optionally comprised within a collagen hydrogel (deposited on said coated scaffold); and
- chondrocytes that are comprised within an alginate hydrogel (deposited on said coated scaffold);
and is for use in subchondral bone regeneration, in osteochondral regeneration, and/or in the treatment of a subchondral bone defect or an osteochondral defect. In other terms, such a biomaterial is particularly well-suited for the treatment of defects affecting both the bone and the cartilage.

In still another preferred embodiment according to the invention, the biomaterial according to the invention comprises or consists of:
- the nanofibrous scaffold made of polymers that is coated with at least one layer pair consisting of a layer of polyanions and a layer of polycations; and
- chondrocytes that are comprised within an alginate hydrogel (deposited on said coated scaffold);
and is for use in cartilage regeneration, and/or in the treatment of a cartilage defect.

The present invention also relates to the use of the biomaterial according to the invention in the field of maxillofacial surgery.

The invention further provides a method for treating a bone and/or cartilage defect, comprising the step of implanting the biomaterial according to the invention in an individual in need thereof.

In the frame of the present invention, the individual and/or patient to be treated preferably is a human individual and/or patient. However, the biomaterials according to the invention also find use in the field of veterinary medicine.

### FIGURES

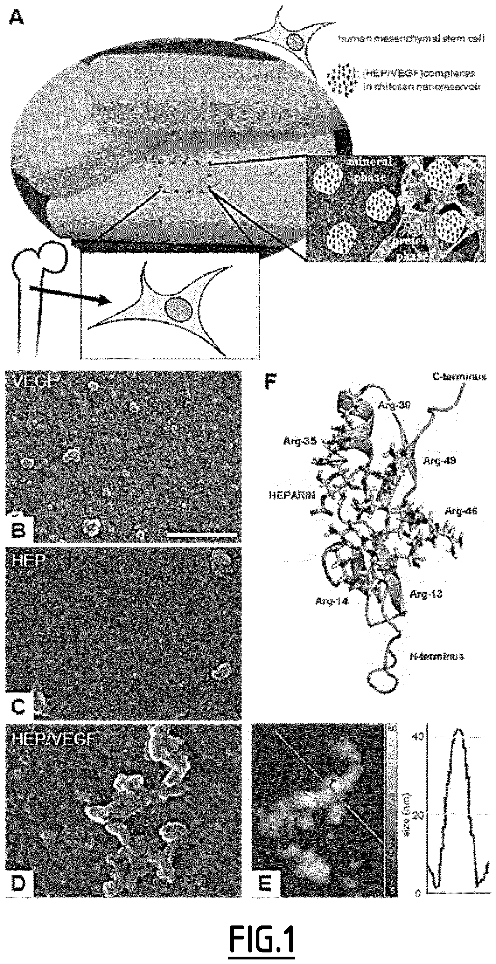

**Figure 1****.** Third-generation bone substitutes nano-functionalized with angiogenic molecules.
   (A) The combination of angiogenic nanoreservoirs, human mesenchymal stem cells and biphasic bone substitutes used in the study.
   (B-D) SEM pictures of VEGF (B), HEP (C) and HEP/VEGF complex (D) after deposition on glass. Scale bar: 500 nm.
   (E) The HEP/VEGF complex shown in (C), representative of the complexes screened (n = 6), was also acquired using atomic force microscopy (AFM). False colours indicate depth, as for the side color bar.
   (F) Molecular modelling of the HEP/VEGF complex. VEGF is displayed as homology model (green); HEP is displayed as stick model; yellow indicates sulphur moieties.

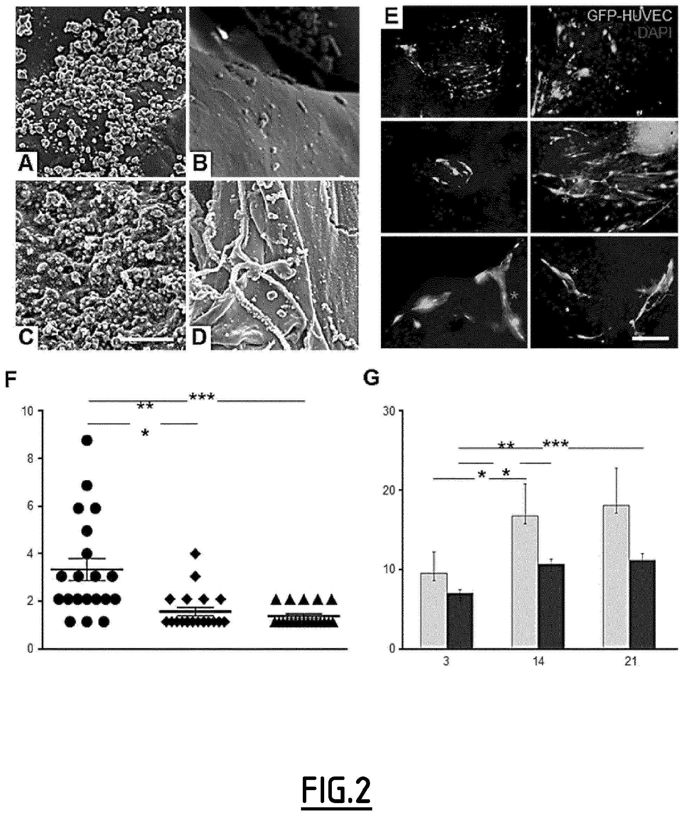

**Figure 2****.** Nano-functionalization of the Anatartik® sponge with HEP/VEGF-NRs and its effect on endothelial cells.
   (A-D) SEM pictures of the Antartik® sponge deposited with empty (NF-) (A,B) or HEP/VEGF- (C,D) NRs. The NRs were found on both the mineral (A,C) and the protein (B,D) constituents of the sponge. Scale bar: 6 µm.
   (E) Fluorescence micrographies of GFP-HUVECs on the Antartik® sponge deposited with NF- (top micrographies), HEP/BSA- (middle micrographies) and HEP/VEGF-NRs (lower micrographies) after 21 days of culture (E). Nuclei counterstained with DAPI. Scale bar: 150 µm.
   (F) Number of GFP-HUVECs found organized in vessel-like structures (red asterisk in E) on NF- (right), HEP/BSA- (middle) and HEP/VEGF-NRs (left) bone substitutes. Bars represent mean ± SEM (n = 20 per condition); ***: p < 0.001.
   (G) Reduced Alamar Blue® was assessed (in %) for the cells seeded on bone substitutes either deposited with NF- (grey bars) or HEP/VEGF-NRs (black bars), after 3, 14 and 21 days of culture. Values expressed as mean ± SEM (n = 4). *: p < 0.1; ***: p < 0.01.

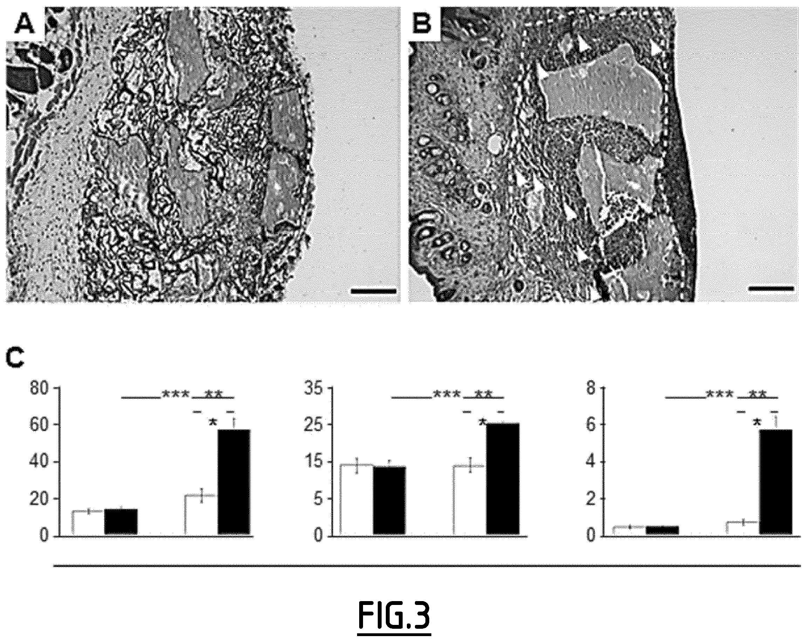

**Figure 3****.** Host vascular infiltration in bone substitutes subcutaneously implanted in nude mice.
   (A-B) H&E staining of Antartik® biphasic sponge (delimited by the yellow frames), either deposited with empty (NF-) (A) or with HEP/VEGF- (B) NRs, at 12 dpi. White arrowheads indicate blood vessels within the bone substitute.
   (C) Quantitative analysis of the blood vessels found within the implanted bone substitute. The average number (left) and the average diameter (middle) of the blood vessels, together with the average surface covered by the blood vessels (right) found in the explanted bone substitutes are given at 4 (left bars) and 12 dpi (right bars). All values are expressed as mean ± SEM of at least 5 images/section, 4 sections/sample; ***: p ≤ 0.01. Scale bars in A, B: 200 µm.

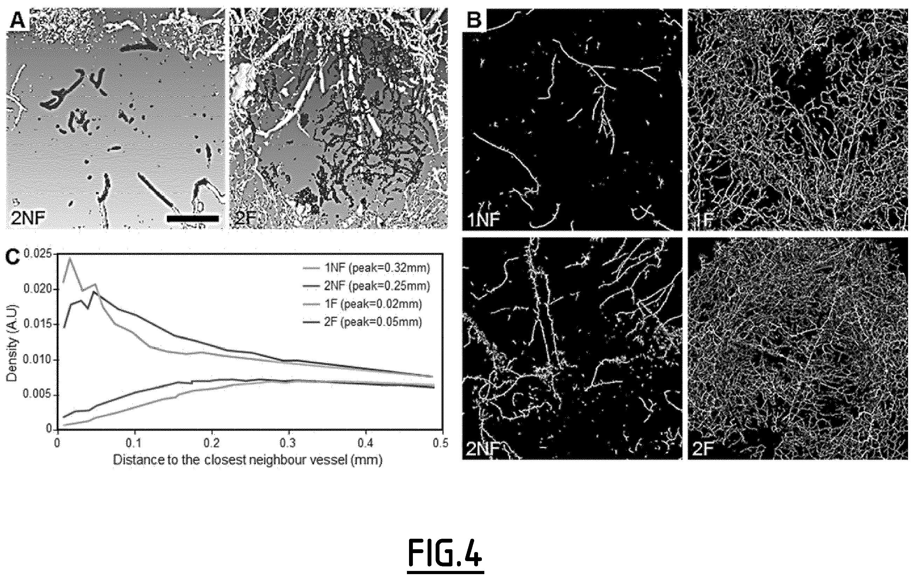

**Figure 4****.** Quantitative analyses of the host vasculature infiltration in the bone substitutes implanted in critical size calvarial bone defect mouse models.
   (A) Micro-CT scans of two representative bone substitutes, either deposited with NF-NRs (2NF) or with HEP/VEGF-NRs (2F). All vessels are shown; the vessels within the bone substitutes are colored in red. Scale bar: 1 mm.
   (B) Tridimensional reconstruction of the vascular network found in 4 representative bone substitutes, either deposited with empty NF-NRs (1NF, 2NF) or with HEP/VEGF-NRs (1F, 2F). Skeletons of the segmented images are represented. Lighter colours display vessels of larger size.
   (C) Relative density of the host vascular network within the bone substitutes shown in B, represented as the average distance to the closest neighbor vessel.

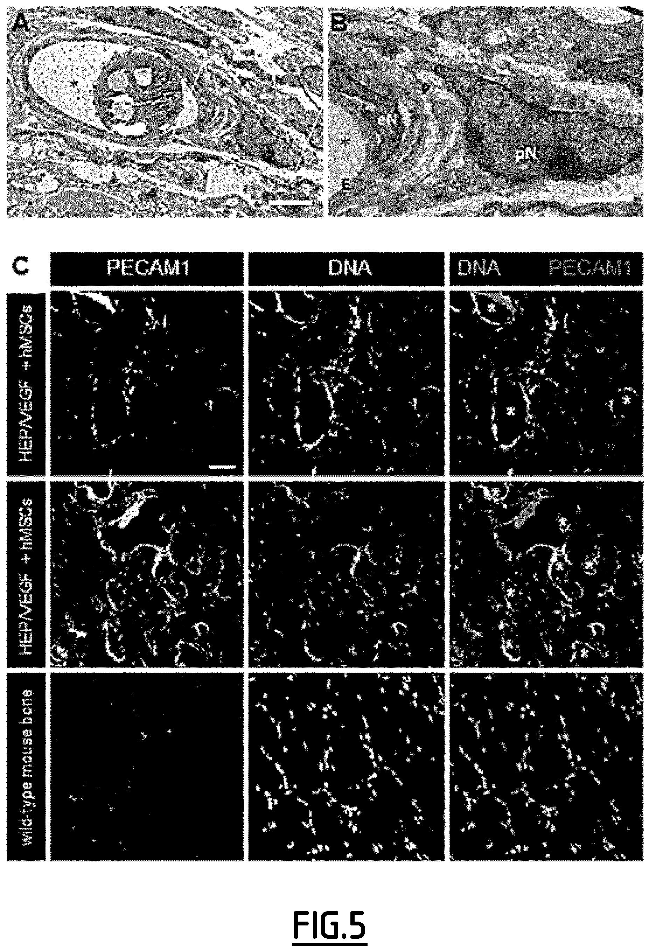

**Figure 5****.** Human MSCs contributed to the vascularization of the bone substitutes subcutaneously implanted in nude mice.
   (A,B) Ultrastructural view of a blood vessel found within the HEP/VEGF-NRs bone substitute, as transverse section (A). The presence of a mural cell (a pericyte, red asterisks) enveloping the blood vessel suggests the good functionality of the blood vessels that infiltrated the active bone substitute (yellow insert in A; B). No blood cells could be seen as the Microfil® MV-122 contrast agent was perfused, which can be observed in the lumen of the vessel (blue asterisks). E: endothelial cell; eN: nucleus of the endothelial cell; P: pericyte; pN: nucleus of the pericyte. Scale bars: 5 µm in A, 2 µm in B.
   (C) The presence of cells of human origin within the HEP/VEGF-NRs bone substitutes implanted subcutaneously was revealed by immunohistochemistry using an antibody specific for human PECAM1 (C, top panels). The same specimen was also subject to immunohistochemistry using an antibody with cross-reactivity for both human and mouse PECAM1 (C, mid panel). In the lower panel, the anti-human PECAM1 antibody was used on a control mouse bone (C, lower panel). Scale bar: 200 µm.

### EXAMPLES

### MATERIALS AND METHODS

### Deposition of the VEGF/HEP complex

Drops of HEP (500 µg ml⁻¹ in 20 mM / 0.15 mM Tris/NaCl, pH 6.8), VEGF (200 µg ml⁻¹ in 20 mM / 0.15 mM Tris/NaCl, pH 6.8) or HEP/VEGF (500 µg ml⁻¹ / 200 µg ml⁻¹) solutions (Sigma-Aldrich, Saint-Quentin-Fallavier, France) were laid on cover glass and dried. Salt crystals from buffer were solubilised in deionized water, by 2 rinsing steps of 5 min each. This deposition procedure was repeated 6 times to increase the quantity of material.

### Scanning electron microscopy (SEM) and atomic force microscopy (AFM) study

To analyze the formation of HEP/VEGF-NRs on the bone substitute, samples were fixed with 4% paraformaldehyde (PFA) for 10 min at 4 °C. After dehydration, the specimens were observed by mean of SEM (either with Hitachi TM1000 or FEG Sirion XL; FEI) in conventional high vacuum mode with a secondary electron detector. A commercial stand-alone AFM microscope Solver Pro (Nt-Mdt Inc., Moscow, Russia) was used to acquire AFM images. Tapping imaging mode was used, with NSG 10 cantilever with a typical resonance frequency of 105 kHz and a spring constant of 2 N m⁻¹. The image resolution was set to 512x512, with a scanning rate of 1 Hz. Images were analyzed using the open source software Gwyddion 2.24 5 (Nečas D, Klapetek P. Gwyddion: an open-source software for SPM data analysis. In: Open Physics (ed^(eds) (2012)).

### Nanoreservoirs deposition on bone substitute

Antartik® sponges (10% collagen I and III, 90% ceramic; Medical Biomat, Vaulx-en-Velin, France) were cut in 5 mm wide fragments and placed in a 96-well plate. They were sterilized with UV light (254 nm, 30 W, distance 20 cm, 30 min exposure). Chitosan-HEP-VEGF-NRs were applied via layer-by-layer deposition, as previously described.^{[29]} Briefly, bone substitutes were alternately dipped in 500 µg ml⁻¹ chitosan solution (Protasan UP CL 113, Novamatrix, Sandvika, Norway) and 500 µg ml⁻¹ / 200 ng ml⁻¹ HEP/VEGF complex solution in 20 mM / 0.15 mM Tris/NaCl, pH 6.8. After each bath, bone substitutes were rinsed three times for 5 min in Tris/NaCl buffer. Before use, bone substitutes were equilibrated in serum-free Dulbecco's modified Eagle's medium (D-MEM).

### Molecular modelling of HEP- VEGF complexes

To prepare the starting structure for simulation, the HEP Sodium salt (CAS 9041-08-1) and VEGF₁₆₅ heparin binding domain coordinates (pdb id: 2VGH, 55amino acids) were extracted from the PDB structure files (Fairbrother WJ, Champe MA, Christinger HW, Keyt BA, Starovasnik MA. Solution structure of the heparin-binding domain of vascular endothelial growth factor. Structure 6, 637-648 (1998)). Partial atomic charges for heparin sodium salt molecule was assigned based on the AM1-BCC method using the antechamber program of AmberTools. The van der Waals and bonded parameters for the heparin were taken from the general amber force field (GAFF)(Wang J, Wang W, Kollman PA, Case DA. Automatic atom type and bond type perception in molecular mechanical calculations. J Mol Graph Model 25, 247-260 (2006)). The AMBER format files of these molecules were then converted to the GROMACS format using the acpype python script. Using Cluspro and Patchdock docking programs, complexes of heparin and VEGF were generated. The coordinate of 6 best complexes was the selected to generate the topology file for each complex to run molecular dynamics (MD) simulations.

MD simulations were performed using GROMACS-4.6 for a period of 30 nsec using explicit water model. The complex was placed in the centre of a cubic periodic box and solvated by the addition of SPC water molecules. The net charge on the system was then neutralized by adding counter ions as required. The energy minimization was done using the steepest descent algorithm. The temperature and pressure were maintained at 300 K and 1 atm using the v-rescale temperature and Parrinello-Rahman pressure coupling method. Production simulations were performed for 30 nsec with a 2 fsec time step. In order to calculate the interaction free energies of the heparin and VEGF complexes, we used the MM/PBSA protocol. The calculations were performed using the g_mmpbsa tool82 which implements the MM-PBSA approach using the GROMACS software packages. The MM/PBSA energies were obtained from samples of 100 snapshots (for each complex) that were extracted from the MD trajectories. All the calculations were done using computational time on BWUniCluster Karlsruhe.

### Cell culture

Green fluorescent protein-expressing HUVECs (EssenBiotech, France) and hMSCs from the bone marrow (PromoCell, Heidelberg, Germany) were cultured in the respective complete media (endothelial growth medium; mesenchymal stem cells growth medium, PromoCell) at 37°C in a humidified atmosphere with 5% CO₂. 1.5 x 10⁴ hMSCs were seeded on each Antartik® sponge fragment deposited or not with HEP/VEGF-NRs, and cultured for 7 days in mesenchymal stem cells growth medium. After 7 days, 5.5 x 10⁴ GFP-HUVECs were seeded on the same samples and cultured in a medium consisting of half hMSCs growth medium, and half endothelial growth medium. Bone substitute fragments seeded with cells were cultured for a total of 21 days, and then processed according to downstream applications.

### Cell biocompatibility analysis

Alamar Blue® (Thermo Fischer Scientific, Waltham, MA, USA) was used to assess cell metabolic activity over time (n = 4). At 3, 14 and 21 days after GFP-HUVECs seeding, cells were incubated in 10% Alamar Blue® in D-MEM without phenol red (Lonza, Levallois-Perret, France) at 37 °C and 5% CO₂. After 4 hours, the supernatant was transferred to 96-well plates and the absorbances at 570 and 595 nm were measured in a Multiskan FC plate reader (ThermoFischer Scientific, Illkirch-Graffenstaden, France) to determine the percentage of reduced Alamar Blue®.

### Immunofluorescence

Bone substitutes seeded with cells were fixed with 4% PFA for 10 min at 4 °C and rinsed with PBS, then demineralized and embedded in paraffin for 4 µm serial sections. Rabbit anti- human PECAM1/CD31 (Microm, Brignais, France) and non species-specific anti-PECAM1/CD31 (Abcam, Paris, France), then secondary antibody raised against rabbit antibodies and coupled with Alexa 594 fluorochrome (Molecular Probes, Life Technologies, ThermoFisher Scientific), diluted 1:200 in PBS 1% BSA, were used. After multiple rinses in PBS, samples were incubated 10 min in 200 nM 4',6-diamidino-2-phenylindole (DAPI; Sigma-Aldrich), for nuclear counterstaining. Specimens were observed with a LEICA DM4000B epifluorescence microscope (Leica Microsystems, Nanterre, France).

### Implant of bone substitutes in nude mice

All procedures regarding animals and tissues were designed in agreement with the recommendations of the European Union (2010/63/EU), and were performed by investigators authorized, by the Prefecture du Bas-Rhin. All experiments were performed in the Animalerie Centrale de la Faculté de Médecine de Strasbourg with approval number C-67-482-35 from the Veterinary Public Health Service of the Prefecture du Bas-Rhin, representing the French Ministry of Agriculture, Department of Veterinary Science. Nude male mice (Crl: NIH-Foxn1^{nu}; Charles River, L'arbresle, France), 6 weeks old, were anesthetized with an intra-peritoneal injection of 100 mg kg⁻¹ of ketamine (VIRBAC Santé Animale; Centravet, Nancy, France) mixed with 10 mg kg⁻¹ of Xylazine (Rompun® 2%, VIRBAC Santé Animale; Centravet, Nancy, France) and placed on a heating plate kept at 37°C. Five 5 days before implant, each bone substitute was seeded with 1.0 x 10⁵ hMSCs. For subcutaneous implant, dorsal skin incision was performed and bone substitute (diameter = 5 mm) was placed between the skin and the muscle below. Incisions were sutured with resorbable material and mice were kept under observation for the whole experimentation time. After either 4 (n = 6) or 12 (n = 12) dpi, implanted mice were sacrificed with an intra-peritoneal injection of a lethal dose of ketamine. For implant in bone critical size defect, skin incision was performed, calvaria (parietal zone of the skull) was drilled using a sterile round burr (500 µm deep and 5 mm in diameter) and bone substitute (diameter = 5 mm) was placed in the defect. Incisions were sutured with resorbable material and mice were kept under observation for the whole experimentation time. Mice (n = 12) were sacrificed 12 dpi and explants were subject to histological, TEM and/or micro-CT assessments.

### Hematoxylin-eosin (HE) histological staining

For HE staining, subcutaneous explants were fixed with 4% PFA, demineralized and embedded in paraffin for 7 µm serial sectioning. Samples were then subject to HE staining and observed on a Leica DM4000B microscope (Leica Microsystems). For quantitative analysis of the vascularization, imageJ software was used. Number, mean size and total area of the blood vessels found in the explants were analysed. At least, 5 images per section, 4 sections per sample were analyzed.

### Microangiography and quantitative analysis of the vasculature

Twelve days after subcutaneous implant, mice were subject to deep general anaesthesia (sodium Pentobarbital 120 mg Kg⁻¹). After opening the thoracic cage, an infusion needle was placed in the left ventricle. Mice were in turn perfused (rate of 2 ml min⁻¹) with heparin (50 U ml⁻¹; to purge the cardiovascular system), 4% PFA, PBS and radiopaque silicone rubber (Microfil® MV-122, Flow Tech Inc.). After perfusion, the heart was clamped to avoid leaks of the contrast agent; mice were then placed at 4 °C, overnight to allow the polymerization of the contrast agent. Explants were then post-fixed in 4% PFA for 48 hours and then demineralized with Ethylenediaminetetraacetic acid (EDTA, 15%, PH=7,4) for 1 week at 37 °C under constant slow agitation. The explants were mounted in 1% agar, in order to avoid any movement of the sample during micro-CT acquisition. The tomography experiments were carried out using the micro-CT X-ray system nanotom® m (GE Sensing & Inspection Technologies GmbH, Wunstorf, Germany) equipped with a 180 kV - 15 W high-power nanofocus tube with a tungsten transmission target. The X-ray micro-CT was so performed with an isotropic pixel size of 5 mm² and a field of view of about 15.4 x 12.0 mm². For each measurement, the sample was irradiated by X-rays of 60 kV acceleration voltage and 310 mA beam current. At each rotation angle position six images were acquired and averaged to a projection. 1700 projections over 360° resulted in a total scan duration of about 100 min.

The data acquisition and reconstruction were performed with the phoenix datos|x 2.0 software (phoenix|x-ray, GE Sensing & Inspection Technologies GmbH, Wunstorf, Germany).

Grey level images obtained from micro-CT scan were segmented into two classes to distinguish the voxels within the vessels from those outside. For each image, manually tuned threshold was applied to intensity levels. A structuring element 10 µm wide was applied to each image in order to de-noise the segmentation. Then, the connected sets of voxels smaller than 20 µm³ were erased. After segmentation, the resulting vessels were sketched as skeletons, where a skeleton is defined as the ordered set of the points that defines both the center line and the local radius of a vessel-like shape, as for the algorithm previously described.

### Transmission electron microscopy (TEM)

After the micro-CT scan, explants at 12 dpi were fixed in 2.5% glutaraldehyde and 2.5% PFA in 0.1 M cacodylate buffer, pH 7.4. The samples were post-fixed in 1% osmium tetroxide, dehydrated, conditioned in propylene oxide and embedded in Epon 812, Spurr's epoxy resin (Electron Microscopy Sciences, Ft. Washington, PA). Semithin sections (2 µm) and ultrathin sections (70 nm) were prepared on an Leica Ultracut UCT ultra microtome (Leica Microsystems), contrasted with uranyl acetate (Laurylab, Brindas, France) and lead citrate (Euromedex EMS, Souffelweyersheim, France) and examined at 70 kv with a Morgagni 268D electron microscope (FEI-Phillips, ThermoFisher Scientific). Digital images were captured using a Mega View III camera (Soft Imaging System, Münster, Germany).

### Statistical analysis

Statistical analysis was carried out with BioStatGV (Sentiweb, France) and Prism5 (GraphPad, La Jolla, CA, USA). Alamar Blue metabolic assay, histological data and microangiography data were analyzed with unpaired, two-tailed Student's *t*-test. When variance was found different between sets of data, then the Welch correction was applied.

### Example 1: Design and modelling of a bone substitute nano-functionalized with angiogenic complexed molecules

In order to promote the vascularization of a transplanted bone substitute, the inventors functionalized the FDA-approved Antartik® sponge with the NR technology (Figure 1 A)(Mendoza-Palomares C, et al. Smart hybrid materials equipped by nanoreservoirs of therapeutics. ACS Nano 6, 483-490 (2012); Eap S, et al. Collagen implants equipped with 'fish scale'-like nanoreservoirs of growth factors for bone regeneration. Nanomedicine (Lond) 9, 1253-1261 (2014); Schiavi J, et al. Active implant combining human stem cell microtissues and growth factors for bone-regenerative nanomedicine. Nanomedicine (Lond) 10, 753-763 (2015); and Eap S, et al. A living thick nanofibrous implant bifunctionalized with active growth factor and stem cells for bone regeneration. Int J Nanomedicine 10, 1061-1075 (2015)). Heparin is critical for the angiogenic activity of VEGF, binding the growth factor through the heparin-binding domain located in its carboxy-terminal domain (Fairbrother WJ, Champe MA, Christinger HW, Keyt BA, Starovasnik MA. Solution structure of the heparin-binding domain of vascular endothelial growth factor. Structure 6, 637-648 (1998); Krilleke D, Ng Y-Shan E, Shima David T. The heparin-binding domain confers diverse functions of VEGF-A in development and disease: a structure-function study. Biochemical Society Transactions 37, 1201-1206 (2009)). To maximize the angiogenic effects through NR-mediated cell contact-dependant delivery, the inventors first investigated the suitability of the HEP/VEGF₁₆₅ complex to functionalize the bone substitute. Heparin, VEGF or a complex thereof was deposited drop-wise on a glass coverslip, and visualized by scanning electron microscopy (Figures 1B-D). Complexed HEP/VEGF₁₆₅ formed large agglomerates (60.0 ± 15.4 nm), as confirmed by AFM imaging (Figure 1E).

To understand why complexed HEP/VEGF aggregates had such a large size, the inventors modelled the molecular interaction between HEP and VEGF₁₆₅ using molecular dynamics simulations. Heparin is a linear sulphated polysaccharide, with high negative charge, while the surface electrostatic potential of the HEP-binding domain of VEGF₁₆₅ is positively charged (Ono K, Hattori H, Takeshita S, Kurita A, Ishihara M. Structural features in heparin that interact with VEGF165 and modulate its biological activity. Glycobiology 9, 705-711 (1999)). Docking simulations of the HEP model to the NMR model of VEGF₁₆₅ were therefore performed using the sulphate anions of HEP and the arginine (Arg) residues of VEGF as a guide to model the molecular interaction. Most of the positive Arg residues are either clustered in the central domain of VEGF₁₆₅ (Arg35, Arg39, Arg46, Arg49) or within a loop in its N-terminal domain (Arg13, Arg14). These regions, according to the calculated electrostatic potential of the solvent accessible surface, represent the binding site for a high negatively charged molecule, such as HEP. The HEP/VEGF complex models generated using molecular docking were subjected to steepest decent energy minimization, followed by equilibration and a 30 nsec molecular dynamics production run. The complex models were then ranked according to their energy, by means of the Molecular Mechanics Poisson-Boltzmann Surface Area (MM/PBSA) method. The resulting HEP/VEGF complex structure with the lowest energy (Figure 1F) clearly indicates that the net charge plays a significant role in the affinity of HEP (stick model in Figure 1F) to VEGF₁₆₅ (homology model in Figure 1F). The sulphate groups of HEP (yellow in Figure 1F) strongly interact with the side chains of both the Arg residues mentioned above and the leucine 17 and threonine 47 residues of VEGF₁₆₅, stabilizing the HEP/VEGF complex and likely promoting the formation of large agglomerates.

### Example 2: Bone substitutes nano-functionalized with HEP/VEGF-NRs improve the organization of endothelial cells in vitro

Heparin/VEGF complexes were integrated into chitosan NRs and deposited on the Antartik® sponge. The functionalized bone substitutes were observed at the SEM and compared to chitosan only-NRs (non-functionalized NRs: NF-NRs). After 6 cycles of deposition, we observed a homogeneous distribution of either HEP/VEGF- or NF-NRs, on both the mineral (Figures 2A,C) and the collagen (Figures 2B,D) portions of the bone substitute. To investigate the pro-angiogenic effects of the nano-functionalized Antartik® bone substitute, either HEP/VEGF-NRs, HEP/BSA-NRs or NF-NRs were deposited on fragments of bone substitutes that were in turn seeded with hMSCs and green fluorescent protein (GFP)-expressing Human Umbilical Vein Endothelial Cells (GFP-HUVECs). The organization of the endothelial cells was monitored after 21 days of culture. In the presence of HEP/VEGF-NRs, GFP-HUVECs organized in stretches of cells, resembling vessel-like structures (red asterisks in Figure 2E), which in many cases showed a lumen. On the contrary, in the presence of either NF-NRs or HEP/BSA-NRs (Figure 2E), the endothelial cells remained mostly distributed as single cells on the graft. The vessel-like structures were assessed by means of number of GFP-HUVECs aligned in a continuous stretch. On nanoactive scaffolds, these were on average 3.4 ± 0.5 cells long, while on non functionalized scaffolds or on scaffolds functionalized with non-angiogenic HEP/BSA, these were 1.2 ± 0.1 and 1.4 ± 0.2 cells long, respectively (p ≤ 0.001) (Figure 2F). Since the presence of HEP/BSA-NRs produced results similar to those found for NF-NRs, we concluded that the capacity of the nanoactive bone substitute to promote the organization of endothelial cells *in vitro* depended on the presence and cellular availability of the HEP/VEGF complex. We also assessed the metabolic activity of the cells seeded on non-functionalized scaffolds, and compared it with that of cells seeded in the presence of HEP/VEGF-NRs, by means of alamar blue assay. In both conditions, the reduction of the alamar blue increased from day 0 to day 21 (Figure 2G), suggesting that no cytotoxic cues affected the cells. No significant differences were observed between NF- and HEP/VEGF-NRs along the culture period considered. However, while the cells cultured with NF-NRs showed an abrupt metabolic increase between day 3 and day 14 (p ≤ 0.1), the cells cultured in the presence of HEP/VEGF-NRs showed a more constant metabolic rate (p ≤ 0.05 and p ≤ 0.01, for day 3-14 and day 14-21, respectively). These data suggest that on non-functionalized scaffolds, the cells may proliferate more than in the presence of HEP/VEGF-NRs.

### Example 3: Nanoactive bone substitutes implanted subcutaneously recruited host vasculature

Bone substitutes functionalized with HEP/VEGF-NRs improved the organization of endothelial cells *in vitro,* eliciting the formation of vessel-like structures. Therefore, the inventors assessed if and how the angiogenic nanoactive bone substitute could effectively trigger vasculoneogenesis *in vivo.* The inventors subcutaneously implanted either HEP/VEGF-NRs or NF-NRs bone substitute in the dorsal of nude mice. Prior to implant, all bone substitutes were seeded with hMSCs and cultured for 7 days. Bone substitutes were explanted from mice after 4 or 12 days post-implant (dpi), and their degree of vascularization was evaluated at the histological levels. In general, explanted nanoactive bone substitutes showed a better recruitment of host blood vessels compared to NF ones (white arrowheads in Figures 3A,B). The quantification of the blood vessels found in the core of the bone substitutes showed a significant increase only in the presence of HEP/VEGF-NRs (14.8 ± 1.7 mm⁻¹ and 61.0 ± 12.9 mm⁻¹ at 4 and 21 dpi, respectively; p = 0.002), compared to NF bone substitutes (13.2 ± 2.74 mm⁻¹ and 22.6 ± 8.0 mm⁻¹ at 4 and 12 dpi, respectively) (Figure 3C). No significant differences were observed at 4 dpi in the diameter of the blood vessels recruited (17 ± 1 µm *vs.* 18 ± 2 µm, in HEP/VEGF-NRs and NF-NRs bone substitutes, respectively) (Figure 3E) and in the relative vessel area (below 1% in either condition) (Figure 3E). The picture changed quite dramatically at 12 dpi, when both the size and the relative area of the host blood vessels found in the HEP/VEGF-NRs bone substitutes increased of 1.5- (p = 0.002) and 8-fold (p = 0.0003), respectively, while no differences were observed in the NF-NRs bone substitutes (Figure 3E).

These results show how the presence and prolonged availability of the HEP/VEGF complex thanks to the NR technology increased the number and the size of the blood vessels recruited from the host in the core of the bone substitute, suggesting that a better functionality of the graft could also be expected.

### Example 4: Nanoactive bone substitutes promoted vasculoneogenesis in a critical size calvarial bone defect mouse model

Eventually, we tested the effect of the nanoactive HEP/VEGF bone substitute in a mouse model of critical size bone defect. A portion of the skull roof was drilled out in mice (induced calvarial bone defect), and filled with either the HEP/VEGF-NRs or the NF-NRs bone substitute. At 12 dpi, implanted animals were perfused with a radiodense rubber contrast agent and bone substitutes were then explanted and subject to micro-CT scan. 3D micro-angiographies of the transplanted mice (3D rendering of micro-CT scan images) were prepared in order to show the influence elicited by the presence of the nanoactive HEP/VEGF complexes on the recruitment of host vasculature, compared to empty NRs (Figure 4A). The newly formed vessels penetrated intimately the HEP/VEGF-NRs bone substitutes, covering virtually the entire volume of the implant (Figure 4B, left panels). On the contrary, the host vasculature colonized the NF-NRs bone substitutes sparsely (Figure 4B, right panels). Remarkably, when using Standard Euler distances to generate the distance maps to the closest neighbouring vessel in the segmented images, it was seen that both the trend of the curves (Figure 4C) and the mode of the distributions (0.32, 0.25 and 0.05 mm for 1NF, 2NF and 3NF, respectively; 0.06, 0.02 and 0.05 mm for 1F, 2F and 3F, respectively) clearly indicated that the average distance of any point to the nearest neighbouring vessel is smaller in the nanoactive bone substitutes compared to the NF one (p = 0.0423). The analyses also revealed that the vascular density in NF bone substitutes was 0.75 ± 0.99%, where in HEP/VEGF-NRs bone substitutes was 2.8 ± 0.76% (p = 0.0167), which is almost 4 times higher.

In summary, these data indicated that the nano-functionalization of the bone substitutes resulted in a denser cloud of new vessels formed inside the treated calvarial bone defect, which is an essential precondition for the bone regeneration.

### Example 5: Human MSCs seeded on the bone substitutes contributed to vasculoneogenesis

The vessels observed within the bone substitutes in the micro-angiographies look well connected to the surrounding vessels. Also, the implanted animals did not suffer bleeding, and when perfused with the contrast agent, they did not show any leakage of the agent in the surrounding bone, which could point towards a sub-functional vasculature. In order to have a closer look at the morphology of the newly formed vessels, we analyzed them at the ultrastructural level. The vessels found within the bone substitutes were characterized by the presence of tightly connected endothelial cells of normal morphology, surrounded by mural cells that provide structural stability to the vessel (Figures 5A and 5B). The functionality of the blood vessels that are found within the bone substitute after implant, together with the speed at which they form are both of crucial importance to avoid the necrosis of the cellular component of the transplanted bone substitutes. Both functionalized and non-functionalized bone substitutes were seeded with hMSCs before implant, in order to regrow the missing bone. Mesenchymal stem cells are multipotent stem cells known to be able to differentiate in many cell types, like osteoblasts, adipocytes, chondrocytes and even neurons. Studies also suggested that MSCs could efficiently differentiate in endothelial cells, at least *in vitro.* Therefore, the inventors investigated whether the hMSCs seeded on the bone substitute contributed to the formation of the blood vessels found within the implant. By means of immunohistochemistry, the inventors detected several endothelial cells positive to anti-human-PECAM1 antibody (Figure 5C, top panels), which were part of the endothelium of the newly formed blood vessels within the bone substitute (white asterisks in Figure 5C). Only a portion of these cells originated from the grafted hMSCs, as shown by the immunohistochemistry using an antibody that cross-reacted with both human and mouse PECAM1 (Figure 5C, mid panels). As expected, no signal was detected using the anti-human-PECAM1 antibody on a control mouse bone (Figure 5C, lower panels). These results indicate that the formation of new blood vessels within the implanted bone substitutes depend on the simultaneous presence of both the NRs and the hMSCs. It was therefore concluded that the cell contact-dependent release of angiogenic growth factors over a prolonged time combined to the active differentiation of hMSCs in cells of the endothelium synergistically contribute to the functional vascularization that infiltrate the nano-functionalized bone substitutes.

The present invention is based on the nano-functionalization of the bone substitute material, which allows cell contact-dependent release of VEGF. In order to maximise the angiogenic effect, VEGF₁₆₅ was complexed with HEP. The collected evidences showed that the HEP/VEGF complex formed larger aggregates than VEGF alone (Figure 1B-E), as a result of the chemical-physical interaction with HEP, as anticipated by computer modelling (Figure 1F). Nanoreservoir-mediated functionalization of the bone substitute is extremely advantageous, as it effectively overcomes the side effects associated with a high local dose of VEGF. Moreover, since VEGF₁₆₅ has a short half-life of approximately 90 min, when exposed to the extracellular environment, within the chitosan NRs it does not undergo degradation, and is therefore available to the cells for a prolonged time. In this work, we showed how HEP/VEGF-NRs induced the organization of endothelial cells in vessel-like structures *in vitro* (Figures 2E,F) and could improve the vascularization of a clinically used biphasic bone substitute, implanted either subcutaneously (Figures 3A-C) or in a critical size calvarial bone defect (Figures 4A-C). Although the spatial distribution of the host blood vessels in one NF-NRs bone substitutes implanted in the calvarial defect was found similar to that found for HEP/VEGF-NRs bone substitutes, the vascular density was lower (1.9%) compared to nanoactive bone substitutes (2.8%). These results probably owe to the average diameter of the blood vessels and could be explained by a difference in the maturity of the vessels, as observed in some histology specimens.

Together with HEP/VEGF-NRs, the Antartik® biphasic sponge was seeded with hMSCs, prior implant. Recently, with the emergence of the third generation biomaterials, the use of adult mesenchymal stem cells as therapeutic agents has struck a great interest for clinical applications. These cells are not only able to differentiate into mature tissues, but also modulate immune reactions, prevent apoptosis and promote angiogenesis, thanks to the secretion of trophic factors. Moreover, mesenchymal stem cells were shown to stabilize newly formed blood vessels, by differentiating in both mural and endothelial cells. The combined use of hMSCs, endothelial cells, and 3D biomaterials, was shown to especially increase the formation of both a vascular network and new bone tissue.

Therefore, the inventors combined the presence of angiogenic NRs with hMSCs onto a clinically used biphasic sponge and observed how these two players could synergistically promote vasculoneogenesis in the Antartik® bone substitute *in vivo.* We showed that, besides an improved recruitment of the endothelial cells from the host, the hMSCs actively contributed themselves to the formation of blood vessels, as several endothelial cells of human origin were found in the vasculature that colonized the bone substitute (Figure 5C).

In summary, thanks to the simultaneous presence of cell contact-dependent sustained release of angiogenic factors and of hMSCs on the Antartik® bone substitutes, the following observations were made: 1) the formation of a functional vasculature, proved by the presence of mural cells also at the core of the large graft (Figures 5A,B); 2) a better vascularization of the bone substitute subcutaneously implanted, in terms of number, size and relative area of the blood vessels found in the presence of VEGF/HEP-NRs and hMSCs (Figures 3A-C); 3) a denser network of blood vessels in the bone substitutes implanted in a calvarial large bone defect (Figures 4A-C); and 4) an active contribution of the hMSCs to vasculoneogenesis (Figure 5C).

As of today, autologous bone grafting (auto-transplant) is the gold standard in the treatment of large bone defects. With ≥ 2 million procedures per year worldwide, it is the second most common tissue transplantation after blood transfusion. However, it has downsides. Bone autografts present more complications than the use of synthetic bone substitutes in terms of infections and they have a higher cost. Allogenic bone grafts are even more expansive than autograft, as they have to be properly treated prior to the clinical use. However, autografts necessarily introduce a second operative site, a longer operating room time and a longer post-operative chronic pain, which contribute to increase the overall costs of this technique. Therefore, advanced functionalized materials are needed to overcome the current limitations (the lack of blood vessels recruitment from host tissues and the necrosis induced at the core of the implanted bone substitute) and to induce the vascularization of the implanted bone substitutes, which is the cornerstone for the regeneration of functional bone tissue.

The present invention thus relates to a nano-functionalized bone substitute based on the combined presence of angiogenic nanoreservoirs and mesenchymal stem cells that aims to improve vasculoneogenesis in critical size bone defect. The results presented here have great relevance from both biomedical and public health perspectives. They showed that this innovative strategy, applied to a bone substitute already used in the clinic, was able 1) to organize endothelial cells in vessel-like structures *in vitro,* 2) to contribute to new vessel formation in subcutaneous implants and 3) to improve the vasculoneogenesis in a critical bone defect mouse model. The nano-functionalized bone substitute of the invention could now replace auto- and allografts in the treatment of large bone defects and, with the due modifications in the composition of the active players (cells and growth factors), could also be used for the regeneration of other tissues.

## Claims

1. A biomaterial comprising a scaffold containing a mineral component, wherein said mineral component comprises at least one calcium phosphate compound,
wherein said scaffold has a surface coated with an interrupted coating made of multilayered droplets, said multilayered droplets being droplets composed of at least one layer pair consisting of a layer of polyanions and a layer of polycations.

2. The biomaterial of claim 1, wherein the scaffold further contains a polymeric component.

3. The biomaterial of claim 1 or 2, further comprising a therapeutic molecule within at least one multilayered droplet or forming at least one multilayered droplet when said therapeutic molecule is charged.

4. The biomaterial of claim 3, wherein the therapeutic molecule is a growth factor selected from the group consisting of: a vascular endothelial growth factor (VEGF), a bone morphogenetic protein (BMP), a transforming growth factor (TGF), a fibroblast growth factor (FGF), a nucleic acid coding therefor, and mixtures thereof.

5. The biomaterial of any one of claims 1 to 4, wherein the calcium phosphate compound is selected from the group consisting of: hydroxyapatite (HA), amorphous calcium phosphate (ACP), monocalcium phosphate anhydrous (MCPA), monocalcium phosphate monohydrate (MCPM), dicalcium phosphate dihydrate (DCPD), dicalcium phosphate anhydrous (DCPA), precipitated or calcium-deficient apatite (CDA), β-tricalcium phosphate (β-TCP), tetracalcium phosphate (TTCP), and mixtures thereof.

6. The biomaterial of any one of claims 2 to 5, wherein the polymeric component is made of a polymer chosen from the group consisting of: poly(ε-caprolactone), collagen, fibrin, poly(lactic acid), poly(glycolic acid), poly(ethylene glycol)-terephtalate, poly(butylenes terephtalate), or co-polymers thereof, and mixtures thereof.

7. The biomaterial of any one of claims 1 to 6, wherein the polycations are chosen from the group consisting of: poly(lysine) polypeptides (PLL), covalently-coupled cyclodextrin-poly(lysine) (PLL-CDs), poly(arginine) polypeptides, poly(histidine) polypeptides, poly(ornithine) polypeptides, Dendri-Graft Poly-lysines (e.g. Dendri-Graft Poly-L-lysines), chitosan, and mixtures thereof.

8. The biomaterial of any one of claims 1 to 7, wherein the polyanions are chosen from the group consisting of: poly(glutamic acid) polypeptides (PGA), poly(aspartic acid) polypeptides, and mixtures thereof.

9. The biomaterial of any one of claims 1 to 8, further comprising living cells.

10. A method for preparing the biomaterial of any one of claims 1 to 9, said method comprising a step of coating a scaffold containing a mineral component and an optional polymeric component with at least one layer pair consisting of a layer of polyanions and a layer of polycations.

11. The method of claim 10, wherein the step of coating with at least one layer pair comprises the following steps:
vii. immersing the scaffold in a solution comprising the polycations;
viii. rinsing the scaffold obtained at the end of step (i);
ix. immersing the scaffold obtained at the end of step (ii) in a solution comprising the polyanions;
x. rinsing the scaffold obtained at the end of step (iii); and, optionally;
xi. repeating step (i) to (iv) for at least a second time; and, optionally;
xii. sterilizing the scaffold obtained at the end of step (iv) or (v).

12. The use of the biomaterial of any one of claims 1 to 9 as a bone substitute.

13. The biomaterial according to any one of claims 1 to 9, for use as a bone and/or cartilage defect filling material, or for use in bone and/or cartilage regeneration.

14. The biomaterial according to any one of claims 1 to 9, for use in the treatment of a bone and/or cartilage defect.
